(19) Europäisches Patentamt
European Patent Office
Office européen des brevets

(11) Veröffentlichungsnummer: **0 045 911**
**B1**

(12) # EUROPÄISCHE PATENTSCHRIFT

(45) Veröffentlichungstag der Patentschrift: **14.12.83**

(21) Anmeldenummer: **81106018.5**

(22) Anmeldetag: **31.07.81**

(51) Int. Cl.³: **C 07 D 209/42,** C 07 D 209/12, C 07 D 209/14, C 07 D 405/12, A 61 K 31/40

(54) **Neue Aminopropanol-Derivate, Verfahren zu ihrer Herstellung und diese Verbindungen enthaltende Arzneimittel.**

(30) Priorität: **08.08.80 DE 3030047**

(43) Veröffentlichungstag der Anmeldung: **17.02.82 Patentblatt 82/7**

(45) Bekanntmachung des Hinweises auf die Patenterteilung: **14.12.83 Patentblatt 83/50**

(84) Benannte Vertragsstaaten: **AT BE CH DE FR GB IT LI LU NL SE**

(56) Entgegenhaltungen:
**EP - A - 0 014 951**
**DE - A - 2 830 211**
**DE - A - 2 925 448**

(73) Patentinhaber: **Boehringer Mannheim GmbH, *Sandhoferstrasse 116, D-6800 Mannheim 31 (DE)***

(72) Erfinder: **Michel, Helmut, Ziegelgasse 2a, *D-6800 Mannheim 31* (DE)**
Erfinder: **Kampe, Wolfgang, Dr.rer.nat, Zedernstrasse 49, D-6805 Heddesheim (DE)**
Erfinder: **Ofenloch, Roland, Hibiscusweg 2, D-6143 Lorsch (DE)**
Erfinder: **Dietmann, Karl, Prof.Dr.med., Eisenacher Weg 75, D-6800 Mannheim (DE)**
Erfinder: **Sponer, Gisbert, Dr.med.vet., Tilsiterstrasse 30, D-6944 Hemsbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Neue Aminopropanolderivate, Verfahren zu ihrer Herstellung und diese Verbindungen enthaltende Arzneimittel

Die vorliegende Erfindung betrifft neue Aminopropanolderivate der allgemeinen Formel I:

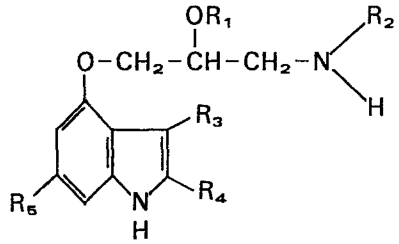

(I)

in welcher

$R_1$ Wasserstoff, eine $C_1$ bis $C_5$ Alkanoyl- oder eine Benzoylgruppe,

$R_2$ eine $C_1$ bis $C_6$ Alkylgruppe oder eine Gruppe

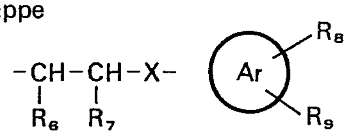

worin

X eine Bindung, eine Methylengruppe oder ein Sauerstoffatom,

Ar einen Phenylrest,

$R_6$ und $R_7$ gleich oder verschieden, jeweils Wasserstoff oder eine $C_1$ bis $C_6$-Alkylgruppe,

$R_8$ und $R_9$ gleich oder verschieden, jeweils Wasserstoff oder Halogen, eine Hydroxygruppe, eine $C_1$ bis $C_5$-Alkanoylgruppe, eine $C_2$ bis $C_4$-Alkenylgruppe, eine $C_2$ bis $C_4$-Alkinylgruppe, eine $C_1$ bis $C_6$-Alkylgruppe, eine $C_1$ bis $C_6$-Alkoxygruppe, eine $C_2$ bis $C_4$-Alkenyloxygruppe, eine $C_2$ bis $C_4$-Alkinyloxygruppe, eine $C_1$ bis $C_6$-Alkylthiogruppe, eine Aminocarbonylgruppe sowie eine Acetamidogruppe bedeuten,

$R_3$ Cyano,

$R_4$ Wasserstoff, eine $C_1$ bis $C_6$-Alkylgruppe oder eine Gruppe $-CH_2-O-R_1$, wobei $R_1$ die obengenannte Bedeutung hat, und

$R_5$ Wasserstoff oder eine $C_1$ bis $C_6$ Alkylgruppe darstellt,

sowie deren pharmakologisch verträgliche Salze.

Da die Verbindungen der allgemeinen Formel I ein asymmetrisches Kohlenstoffatom besitzen, sind ferner Gegenstand der Erfindung die optisch aktiven Formen und racemischen Gemische dieser Verbindungen.

In 2-Position substituierte Indolderivate ähnlicher Struktur sind in der deutschen Offenlegungsschrift Nr. 2830211 beschrieben.

Überraschenderweise hat sich nun herausgestellt, dass die in 3-Position substituierten Verbindungen der allgemeinen Formel I sowie ihre pharmakologisch unbedenkliche Salze im Gegensatz zu obigen bekannten Verbindungen eine ausgeprägte Hemmung der $\beta_1$-Rezeptoren zeigen, also cardioselektiv sind. Sie eignen sich daher zur Behandlung oder Prophylaxe bei Herz- und Kreislauferkrankungen.

Unter einer $C_1$ bis $C_5$-Alkanoylgruppe der Substituenten $R_1$, $R_8$ und $R_9$ sind geradkettige oder verzweigte Gruppen vorzugsweise mit 1 bis 5 Kohlenstoffatomen zu verstehen.

Bevorzugt sind der Formyl-, Acetyl- sowie der Pivaloylrest.

Der Substituent $R_1$ = Benzoylgruppe kann ein- oder mehrfach durch Halogen, Nitro, Alkylgruppen mit 1 bis 4 Kohlenstoffatomen oder Alkoxygruppen mit 1 bis 4 Kohlenstoffatomen substituiert sein.

Die $C_1$ bis $C_6$-Alkylgruppen der Substituenten $R_2$, $R_4$, $R_5$, $R_6$, $R_7$, $R_8$ und $R_9$ sind geradkettige oder verzweigte Gruppen vorzugsweise mit 1 bis 4 Kohlenstoffatomen, wie z.B. der Methyl-, Äthyl-, n-Propyl-, Isopropyl-, n-Butyl-, Isobutyl-, tert.-Butyl- oder n-Hexylrest. Insbesondere kommen jedoch die Methyl-, Äthyl-, Isopropyl- und die tert.-Butylgruppen in Frage.

Von den $C_2$ bis $C_4$-Alkenyl- und Alkinylgruppen der Substituenten $R_8$ und $R_9$ sind die Allyl- und Propagylgruppe bevorzugt.

Die $C_1$ bis $C_6$-Alkoxygruppen der Substituenten $R_8$ und $R_9$ enthalten vorzugsweise 1 bis 4 Kohlenstoffatome, wie z.B. die Methoxy-, Äthoxy-, Propoxy-, Butoxy- oder Pentoxygruppe. Bevorzugt sind die Methoxy-, Äthoxy- und Propoxygruppe.

Von den $C_2$ bis $C_4$-Alkenyloxy- und Alkinyloxygruppen der Substituenten $R_8$ und $R_9$ sind die Allyloxy- und Propargyloxygruppe bevorzugt.

Unter $C_2$ bis $C_3$ Alkoxycarbonylgruppe des Substituenten $R'_3$ sind die Methoxycarbonyl- und Äthoxycarbonylgruppe zu verstehen.

Unter $C_2$ bis $C_3$-Alkoxycarbimidoylgruppe des Substituenten $R'_3$ sind die Methoxycarbimidoyl- und Äthoxycarbimidoylgruppe zu verstehen.

Von einer $C_1$ bis $C_6$-Alkylthiogruppe der Substituenten $R_8$ und $R_9$ ist der Methylmercaptorest bevorzugt.

Unter Halogen wird im Sinne der Erfindung Fluor, Chlor, Brom und Jod verstanden, vorzugsweise Fluor, Chlor und Brom.

Die Herstellung der Verbindung der allgemeinen Formel I ist dadurch gekennzeichnet, dass man in an sich bekannter Weise eine Verbindung der allgemeinen Formel II:

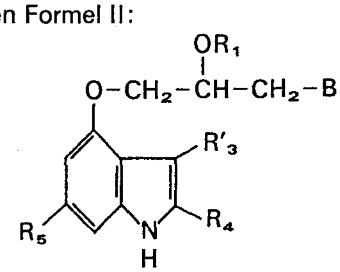

(II)

in welcher

$R_1$, $R_4$ und $R_5$ die obengenannte Bedeutung haben,

B eine reaktive Gruppe darstellt oder zusammen mit $R_1$ einen Valenzstrich bedeutet,

$R'_3$ Carboxyl, $C_2$ bis $C_3$-Alkoxycarbonyl, Aminocarbonyl, Cyano, Oximinomethyl, Formyl oder $C_2$ bis $C_3$ Alkoxycarbimidoyl darstellt,

mit einer Verbindung der allgemeinen Formel (III):

$$R_2-NH-R'_2 \qquad (III)$$

in welcher

$R_2$ die oben angegebene Bedeutung hat und $R'_2$ Wasserstoff oder Benzyl darstellt,

umsetzt und

gegebenenfalls nachträglich in der erhaltenen Verbindungen einen der Reste $R_1$, $R'_2$, $R'_3$ oder $R_4$ nach bekannten Methoden in einen anderen, durch den Anspruch 1 definierten Rest $R_1$, $R_3$ oder $R_4$ überführt und die erhaltenen Verbindungen gewünschtenfalls in ihre pharmakologisch verträglichen Salze überführt.

Reaktive Gruppen in Verbindungen der allgemeinen Formel II sind insbesondere Säurereste, z.B. von Halogenwasserstoffsäuren und Sulfonsäuren. Besonders bevorzugt sind Chloride, Mesyloxy- und Tosyloxyreste.

Die Verbindungen der Formel II können nach den in der deutschen Patentanmeldung P Nr. 3029980.0 beschriebenen Verfahren hergestellt werden.

Die Umsetzungen können ohne Lösungsmittel oder in inerten Lösungsmitteln, wie zum Beispiel Methanol, Äthanol, n-Butanol, Äther, Methylenchlorid, Chloroform, Benzol, Toluol, Essigester, Tetrahydrofuran oder Dioxan durchgeführt werden. Besonders bevorzugt sind Methanol, Äthanol und n-Butanol.

Die erfindungsgemässen Verbindungen der Formel I können in Form eines racemischen Gemisches anfallen. Die Trennung des Racemats in die optisch aktiven Formen geschieht nach an sich bekannten Methoden über die diastereomeren Salze aktiver Säuren, wie z.B. Weinsäure, Äpfelsäure oder Kamfersulfonsäure.

Zur Überführung der Verbindung der allgemeinen Formel I in ihre pharmakologisch unbedenklichen Salze stetzt man diese, vorzugsweise in einem organischen Lösungsmittel, mit der äquivalenten Menge einer anorganischen oder organischen Säure, z.B. Salz-, Bromwasserstoff-, Phosphor-, Schwefel-, Essigs-, Citronen-, Malein-, Benzoesäure um.

Zur Herstellung von Arzneimitteln werden die Substanzen I in an sich bekannter Weise mit geeigneten pharmazeutischen Trägersubstanzen, Aroma-, Geschmacks- und Farbstoffen gemischt und beispielsweise als Tabletten oder Dragées ausgeformt oder unter Zugabe entsprechender Hilfsstoffe in Wasser oder Öl, wie z.B. Olivenöl, suspendiert oder gelöst.

Die erfindungsgemässen neuen Substanzen I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Zitratpuffer, Äthanol, Komplexbildner (wie Äthylendiamintetraessigsäure und deren nichttoxische Salze), hochmolekulare Polymere (wie flüssiges Polyäthylenoxyd) zur Viskositätsregulierung. Feste Trägerstoffe sind z.B. Stärke, Lactose, Mannit, Methylzellulose, Talkum, hochdisperse Kieselsäuren, hochmolekulare Fettsäuren (wie Stearinsäure), Gelatine, Agar-Agar, Kalziumphosphat, Magnesiumstearat, tierische und pflanzliche Fette und feste hochmolekulare Polymere (wie Polyäthylenglykole). Für orale Applikation geeignete Zubereitungen können gewünschtenfalls Geschmacks- und Süssstoffe enthalten.

Bevorzugt im Sinne der vorliegenden Anmeldung sind ausser den in den Beispielen genannten Verbindungen die folgenden:

4-{2-Hydroxy-3-[2-(2-methoxyphenoxy)äthylamino]propoxy}-2-methyl-3-cyanindol

4-{2-Hydroxy-3-[2-(2-allyloxyphenoxy)äthylamino]propoxy}-2-methyl-3-cyanindol

4-{2-Hydroxy-3-[2-(2-acetamidophenoxy)-äthylamino]propoxy}-2-methyl-3-cyanindol

Als gegebenenfalls nachträglich durchzuführende Umwandlung des Substituenten $R'_3$ in Verbindungen der allgemeinen Formel I mit $R_3$ = Cyano kommen beispielsweise

– die Umwandlung einer Formylgruppe in eine Oximinomethylgruppe durch Umsetzen mit Hydroxylamin, und
– die Dehydratisierung einer Aminocarbonyl- oder Oximinomethylgruppe zu einer Cyanogruppe in Frage.

In den folgenden Beispielen wird die Herstellung der erfindungsgemässen Verbindungen näher beschrieben.

*Beispiel 1:*

*4-[2-Hydroxy-3-(N-benzylisopropylamino)-propoxy]-3-formylindol*

3,5 g 4-(2,3-Epoxypropoxy)-3-formylindol werden in 50 ml n-Butanol gelöst, 3,6 g N-Benzylisopropylamin zugegeben und 24 h zum Sieden erhitzt. Nach Erkalten wird abgesaugt. Es werden 3,8 g Titelverbindung (64% d.Th.) vom Schmelzpunkt 107-109°C erhalten.

*Beispiel 2:*

*4-[2-Hydroxy-3-(isopropylamino)propoxy]-3-äthoxycarbonylindol*

11,5 g 4-(2,3-Epoxypropoxy)-3-äthoxycarbonylindol werden in 70 ml Isopropylamin 10 h gerührt, im Vakuum eingeengt und mit 1N-Milchsäure und Äther geschüttelt. Die wässerige Phase wird mit Kaliumcarbonat versetzt, die ausgefallene Base in Essigester/Äther aufgenommen und nach Trocknen über Natriumsulfat und Abdampfen des Lösungsmittels 10,9 g Base der Titelverbindung erhalten. Die Base wird in Essigester gelöst mit der äquivalenten Menge Benzoesäure versetzt und abgesaugt. Man erhält 10,1 g 4-[2-Hydroxy-3-(isopropylamino)-3-äthoxycarbonylindolbenzoat vom Schmelzpunkt 191°C (52% d.Th.).

In analoger Weise erhält man:

| Bezeichnung | Ausbeute (%) | Schmelzpunkt (C°)/ Lösungsm. |
|---|---|---|
| a) 4-[2-Hydroxy-3-(isopropylamino)propoxy]-3-aminocarbonylindolbenzoat aus 4-(2,3-Epoxypropoxy)-3-aminocarbonylindol und Isopropylamin | 25 | 150-152 (Isopropanol) |
| b) 4-[2-Hydroxy-3-(isopropylamino)propoxy]-3-cyanindolbenzoat aus 4-(2,3-Epoxypropoxy)-3-cyanindol und Isopropylamin | 25 | 145-147 (Essigester) |
| c) 4-[2-Hydroxy-3-(isopropylamino)propoxy]-2-hydroxymethyl-3-cyanindol aus 4-(2,3-Epoxypropoxy)-2-acetoxymethyl-3-cyanindol und Isopropylamin | 66 | 178-179 (Essigester) |
| d) 4-[2-Hydroxy-3-(isopropylamino)propoxy]-2-methyl-3-cyanindol aus 4-(2,3-Epoxypropoxy)-2-methyl-3-cyanindol und Isopropylamin | 70 | 158 (Äther) |
| e) 4-[2-Hydroxy-3-(isopropylamino)propoxy]-3-cyan-6-methylindolbenzoat aus 4-(2,3-Epoxypropoxy)-3-cyan-6-methylindol und Isopropylamin | 27 | 189-191 (Essigester) |
| f) 4-[2-Hydroxy-3-(isopropylamino)propoxy]-3-cyan-6-tert.-butylindol aus 4-(2,3-Epoxypropoxy)-3-cyan-6-tert.-butylindol und Isopropylamin | | |
| g) 4-{2-Hydroxy-3-[2-(2-methoxyphenoxy)äthylamino]propoxy}-3-cyanindolbenzoat aus 4-(2,3-Epoxypropoxy)-3-cyanindol und 2-(2-Methoxyphenoxy)äthylamin | 20 | 117-118 (Essigester) |
| h) 4-{2-Hydroxy-3-[2-allyloxyphenoxy)äthylamino]propoxy}-3-cyanindolbenzoat aus 4-(2,3-Epoxypropoxy)-3-cyanindol und 2-(2-Allyloxyphenoxy)äthylamin | 22 | 125-127 (Essigester) |
| i) 4-{2-Hydroxy-3-[2-(2-hydroxyphenoxy)äthylamino]propoxy}-3-cyanindolbenzoat aus 4-(2,3-Epoxypropoxy)-3-cyanindol und 2-(2-Hydroxyphenoxy)äthylamin | 20 | 147-149 (Essigester) |
| j) 4-{2-Hydroxy-3-[2-(3,4-dimethoxyphenyl)äthylamino]-propoxy}-3-cyanindolbenzoat aus 4-(2,3-Epoxypropoxy)-3-cyanindol und 2-(3,4-Dimethoxyphenyl)äthylamin | 25 | 142-143 (Essigester) |

| Bezeichnung | Ausbeute (%) | Schmelzpunkt (C°)/ Lösungsm. |
|---|---|---|
| k) 4-{2-Hydroxy-3-[2-(4-hydroxyphenyl)äthylamino]propoxy}-3-cyanindol-p-nitrobenzoat aus 4-(2,3-Epoxypropoxy)-3-cyanindol und 2-(4-Hydroxyphenyl)äthylamin | 25 | 183 (Methanol) |
| l) 4-{2-Hydroxy-3-[2-(2-allyloxyphenyl)äthylamino]propoxy}-3-cyanindol aus 4-(2,3-Epoxypropoxy)-3-cyanindol und 2-(2-Allyloxyphenyl)äthylamin | 40 | 154-155 (Isopropanol) |
| m) 4-{2-Hydroxy-3-[2-(2-allyloxyphenoxy)äthylamino]propoxy}-3-cyan-6-tert.-butylindol aus 4-(2,3-Epoxypropoxy)-3-cyan-6-tert.-butylindol und 2-(2-Allyloxyphenoxy)äthylamin | 50 | 114-116 (Essigester/Äther) |
| n) 4-{2-Hydroxy-3-[2-(4-hydroxyphenoxy)äthylamino]propoxy}-3-cyanindol aus 4-(2,3-Epoxypropoxy)-3-cyanindol und 2-(4-Hydroxyphenoxy)äthylamin | 30 | 117-119 (Methanol) |
| o) 4-[2-Hydroxy-3-(2-phenoxyäthylamino)propoxy]-3-cyanindol aus 4-(2,3-Epoxypropoxy)-3-cyanindol und 2-phenoxyäthylamin | 50 | 160-162 (Methanol) |
| p) 4-{2-Hydroxy-3-[2-(4-carbamidophenoxy)äthylamino]-propoxy}-3-cyanindol aus 4-(2,3-Epoxypropoxy)-3-cyanindol und 2-(4-Carbamidophenoxy)äthylamin | 20 | 185 (Methanol) |
| q) 4-{2-Hydroxy-3-[2-(2-methylmercaptophenoxy)äthylamino]propoxy}-3-cyanindol aus 4-(2,3-Epoxypropoxy)-3-cyanindol und 2-(2-Methylmercaptophenoxy)äthylamin | 60 | 179-181 (Äthylenglycoldimethyläther) |

*Beispiel 3:*

*4-[2-Hydroxy-3-(isopropylamino)propoxy]-3-formylindolbenzoat*

6,6 g 4-[2-Hydroxy-3-(N-benzylisopropylamino)propoxy]-3-formylindol (Beispiel 1) werden in 150 ml Methanol gelöst, mit 0,5 g 10%ig. Palladiumkohle versetzt und bei Raumtemperatur und 1 bar Wasserstoffdruck hydriert. Nach Filtrieren und Einengen auf ca. 20 ml Volumen gibt man die äquivalente Menge Benzoesäure zu und erhält so nach Absaugen 3,1 g Titelverbindung vom Schmelzpunkt 204-205°C (44% d.Th.).

*Beispiel 4:*

*4-[2-Hydroxy-3-(isopropylamino)propoxy]-3-oximinomethylindolbenzoat*

5,7 g 4-[2-Hydroxy-3-(isopropylamino)propoxy]-3-formylindol werden in 30 ml Äthanol und 30 ml Wasser gelöst, mit 2,2 g Natriumacetat und 1,1 g Hydroxylaminhydrochlorid versetzt und 2 h bei Raumtemperatur gerührt. Nach Abdampfen des Alkohols im Vakuum nimmt man in Essigester auf, versetzt die Essigesterphase mit der äquivalenten Menge Benzoesäure und erhält so 3,3 g der Titelverbindung vom Schmelzpunkt 208-210°C (55% d.Th.).

*Beispiel 5:*

Es wurden Tabletten mit jeweils 50 mg

*4-[2-Hydroxy-3-(isopropylamino)propoxy]-3-cyanindolbenzoat*

gemäss folgender Rezeptur hergestellt:

| | |
|---|---|
| 4-[2-Hydroxy-3-(isopropylamino)-propoxy]-3-cyanindolbenzoat | 50 g |

| | |
|---|---|
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,16 g verpresst.

*Versuchsprotokoll*

β-Blocker werden pharmakologisch dadurch charakterisiert, dass sie die Isoprenalinwirkung blockieren. Dies bezieht sich einmal auf die Isoprenalintachycardie (= $\beta_1$-Wirkung) und zum anderen auf die isoprenalininduzierte Erschlaffung der glatten Muskulatur, was einer Vasodilatation, Blutdrucksenkung und einer Bronchodilatation entspricht (= $\beta_2$-Wirkung). Erwünscht ist jedoch nur die β-blockerinduzierte Hemmung der Isoprenalintachycardie, die therapeutisch der Verhinderung von Herzfrequenzanstiegen bei physischen und psychischen Belastungen entspricht, während die Isoprenalinwirkung auf die glatte Muskulatur trotz der β-Blockergabe möglichst erhalten bleiben soll. Ein solches Wirkspektrum eines β-Blockers wird als cardioselektiv bezeichnet.

Im Tierexperiment wird dieses geprüft, indem die Dosis des β-Blockers gesucht wird, die den Isoprenalineffekt auf die glatte Muskulatur (= Vasodilatation bzw. Blutdrucksenkung) einerseits und die Herzfrequenz andererseits auf die Hälfte begrenzt (= $HD_{50}$).

Ist der Quotient $HD_{50\%\ \text{Blutdrucksenkung}}$ zu $HD_{50\%\ \text{Frequenz}}$ gross, bedeutet dieses, dass für die Hemmung der Isoprenalintachycardie vom β-Blocker relativ kleine Dosen, für die Hemmung der Wirkung an der glatten Muskulatur aber hohe Dosen benötigt werden, also der β-Blocker cardioselektive Eigenschaften aufweist. Je höher also der Quotient, desto cardioselektiver ist die Substanz.

*Versuchsdurchführung:*

Kaninchen werden in Holzkäfige gesetzt und unter Lokalanästhesie ein Katheter in die mittlere Ohrarterie eingeschoben. Über einen elektromechanischen Druckwandler wird fortlaufend der Blutdruck gemessen und daraus bei schnellem Papiervorschub die Herzfrequenz errechnet. Über eine Ohrvene wird zunächst 1 µg/kg Isoprenalin i.v. injiziert, was einen diastolischen Blutdruckabfall um ca. 40 mmHg bewirkt und die Herzfrequenz von ca. 210 auf 320 Schläge/min zur Folge hat. Nach dieser Kontrollinjektion werden die gelösten Prüfsubstanzen der β-Blocker in steigender Dosierung im Abstand von 10 min (Dosen: 0,125 + 0,125 + 0,25 + 0,5 + 1,0 + 2,0 + 4,0 mg/kg i.v.) intravenös verabreicht und danach jeweils erneut die Isoprenalinwirkung analysiert. Sowohl für die Isoprenalinwirkung auf den Blutdruck als auch auf die Herzfrequenz, wird die Dosis des β-Blockers interpoliert, die die Isoprenalinwirkung auf die Hälfte begrenzt (= $HD_{50\%}$).

*Ergebnisse:*

In der folgenden Tabelle werden die Versuchsergebnisse für die erfindungsgemässen Verbindungen zusammengefasst. Als Referenzsubstanzen dienen zwei repräsentative Verbindungen aus der deutschen Offenlegungsschrift Nr. 2830211:

A) 4-[2-Hydroxy-3(isopropylamino)propoxy]-2-cyanindol (vom Anspruch der DE-OS Nr. 2830211 umfasstes Stellungsisomeres zu Beispiel 3b der vorliegenden Anmeldung)

B) 4-[2-Hydroxy-3-(t-butylamino)propoxy]-2-cyanindol (Beispiel 2 aus DE-OS Nr. 2830211)

Die absoluten äquieffektiven Dosen der β-Blockade sind in diesem Fall kein Kriterium für die Güte des Stoffes, alleine der angegebene Quotient ist von Bedeutung.

Alle in der Tabelle angegebenen Daten sind die Mittelwerte von 6 Einzelversuchen.

*Tabelle:*

*Hemmdosen von β-Blockern gegenüber der blutdrucksenkenden bzw. frequenzsteigernden Wirkung von 1 µg/kg Isoprenalin* n = 6, $\bar{x}$

| Substanz | $HD_{50\%\ \text{Bl.}}$ (µg/kg i.v.) | $HD_{50\%\ \text{Fr.}}$ (µg/kg i.v.) | Cardioselektivitätsquotient |
|---|---|---|---|
| A | 2,1 | 1,5 | 1,4 |
| B | 2,6 | 2,6 | 1,0 |
| Beispiel 2b | 34 | 5,5 | 6,2 |
| Beispiel 2c | 3,2 | 7,1 | 0,45 |
| Beispiel 2d | 2,5 | 3,8 | 0,7 |
| Beispiel 2e | 12 | 9,0 | 1,3 |
| Beispiel 2g | 509 | 82 | 6,2 |
| Beispiel 2h | 1143 | 209 | 5,5 |
| Beispiel 2i | 34 | 18 | 1,9 |
| Beispiel 2j | 521 | 71 | 7,3 |
| Beispiel 2k | 201 | 137 | 1,5 |
| Beispiel 2n | 183 | 23 | 8,0 |
| Beispiel 2o | 192 | 53 | 3,6 |
| Beispiel 2p | 343 | 89 | 3,9 |

## Patentansprüche

1. Aminopropanolderivate der allgemeinen Formel (I):

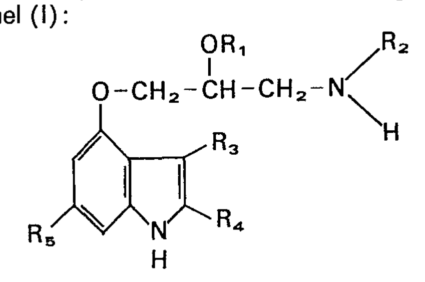

(I)

in welcher

$R_1$ Wasserstoff, eine $C_1$ bis $C_5$-Alkanoyl- oder eine Benzoylgruppe,

$R_2$ eine $C_1$ bis $C_6$ Alkylgruppe oder eine Gruppe

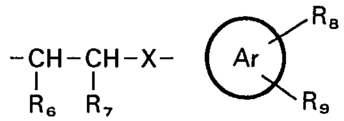

worin

X eine Bindung, eine Methylengruppe oder ein Sauerstoffatom,

Ar einen Phenylrest,

$R_6$ und $R_7$ gleich oder verschieden, jeweils Wasserstoff oder eine $C_1$ bis $C_6$-Alkylgruppe,

$R_8$ und $R_9$ gleich oder verschieden, jeweils Wasserstoff oder Halogen, eine Hydroxygruppe, eine $C_1$ bis $C_5$-Alkanoylgruppe, eine $C_2$ bis $C_4$-Alkenylgruppe, eine $C_2$ bis $C_4$-Alkinylgruppe, eine $C_1$ bis $C_6$-Alkylgruppe, eine $C_1$ bis $C_6$-Alkoxygruppe, eine $C_2$ bis $C_4$-Alkenyloxygruppe, eine $C_2$ bis $C_4$-Alkinyloxygruppe, eine $C_1$ bis $C_6$-Alkylthiogruppe, eine Aminocarbonylgruppe sowie eine Acetamidogruppe bedeuten,

$R_3$ Cyano,

$R_4$ Wasserstoff, eine $C_1$ bis $C_6$-Alkylgruppe oder eine Gruppe $-CH_2-O-R_1$, wobei $R_1$ die obengenannte Bedeutung hat, und

$R_5$ Wasserstoff oder eine $C_1$ bis $C_6$ Alkylgruppe darstellt,

deren optisch aktive Formen, racemische Gemische sowie deren pharmakologisch verträgliche Salze.

2. Verbindungen der allgemeinen Formel (I) gemäss Anspruch 1 sowie deren pharmakologisch verträgliche Salze, in denen $R_1$, $R_3$, $R_4$ und $R_5$ die angegebenen Bedeutungen haben und $R_2$ eine Isopropylgruppe bedeutet.

3. Verbindungen der allgemeinen Formel (I) gemäss Anspruch 1 sowie deren pharmakologisch verträgliche Salze, in denen $R_1$, $R_3$, $R_4$ und $R_5$ die angegebenen Bedeutungen haben und $R_2$ eine Gruppe:

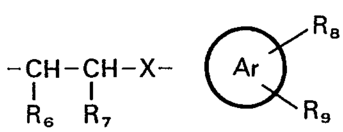

worin

X eine Bindung, eine Methylengruppe oder ein Sauerstoffatom,

Ar einen Phenylrest,

$R_6$ und $R_7$ gleich oder verschieden, jeweils Wasserstoff oder eine $C_1$ bis $C_6$-Alkylgruppe,

$R_8$ und $R_9$ gleich oder verschieden, jeweils Wasserstoff oder Halogen, eine Hydroxygruppe, eine $C_1$ bis $C_5$-Alkanoylgruppe, eine $C_2$ bis $C_4$-Alkenylgruppe, eine $C_2$ bis $C_4$-Alkinylgruppe, eine $C_1$ bis $C_6$-Alkylgruppe, eine $C_1$ bis $C_6$-Alkoxygruppe, eine $C_2$ bis $C_4$-Alkenyloxygruppe, eine $C_2$ bis $C_4$-Alkinyloxygruppe, eine $C_1$ bis $C_6$-Alkylthiogruppe, eine Aminocarbonylgruppe sowie eine Acetamidogruppe bedeuten,

darstellt.

4. Verbindungen der allgemeinen Formel (I) gemäss Anspruch 3 sowie deren pharmakologisch verträgliche Salze, in denen $R_6$, $R_7$, $R_8$, $R_9$ sowie Ar die angegebenen Bedeutungen haben und X eine Valenzbindung darstellt.

5. Verbindungen der allgemeinen Formel (I) gemäss Anspruch 3 sowie deren pharmakologisch verträgliche Salze, in denen $R_6$, $R_7$, $R_8$, $R_9$ sowie Ar die angegebenen Bedeutungen haben und X ein Sauerstoffatom bedeutet.

6. Verfahren zur Herstellung von Verbindungen der allgemeinen Formel (I):

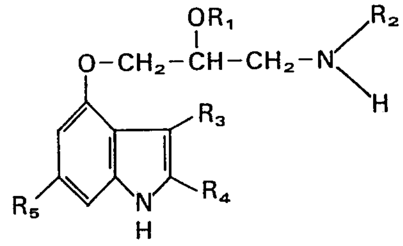

(I)

in welcher

$R_1$ Wasserstoff, eine $C_1$ bis $C_5$-Alkanoyl- oder eine Benzoylgruppe,

$R_2$ eine $C_1$ bis $C_6$ Alkylgruppe oder eine Gruppe

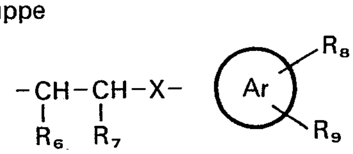

worin

X eine Bindung, eine Methylengruppe oder ein Sauerstoffatom,

Ar einen Phenylrest,

$R_6$ und $R_7$ gleich oder verschieden, jeweils Wasserstoff oder eine $C_1$ bis $C_6$-Alkylgruppe,

$R_8$ und $R_9$ gleich oder verschieden, jeweils Wasserstoff oder Halogen, eine Hydroxygruppe, eine $C_1$ bis $C_5$-Alkanoylgruppe, eine $C_2$ bis $C_4$-Alkenylgruppe, eine $C_2$ bis $C_4$-Alkinylgruppe, eine $C_1$ bis $C_6$-Alkylgruppe, eine $C_1$ bis $C_6$-Alkoxygruppe, eine $C_2$ bis $C_4$-Alkenyloxygruppe, eine $C_2$ bis $C_4$-Alkinyloxygruppe, eine $C_1$ bis $C_6$-Alkylthiogruppe, eine Aminocarbonylgruppe sowie eine Acetamidogruppe bedeuten,

$R_3$ Cyano,

$R_4$ Wasserstoff, eine $C_1$ bis $C_6$-Alkylgruppe oder eine Gruppe $-CH_2-O-R_1$, wobei $R_1$ die obengenannte Bedeutung hat, und

$R_5$ Wasserstoff oder eine $C_1$ bis $C_6$ Alkylgruppe darstellt,

deren optisch aktive Formen, racemische Gemische sowie deren pharmakologisch verträgliche Salze,

dadurch gekennzeichnet, dass man in an sich bekannter Weise eine Verbindung der allgemeinen Formel (II):

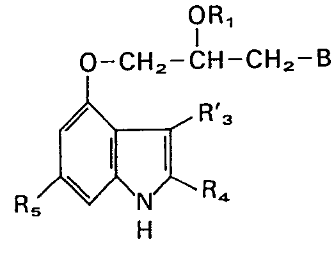

(II)

in welcher

$R_1$, $R_4$ und $R_5$ die obengenannte Bedeutung haben,

B eine reaktive Gruppe darstellt oder zusammen mit $R_1$ einen Valenzstrich bedeutet,

$R'_3$ Carboxyl, $C_2$ bis $C_3$-Alkoxycarbonyl, Aminocarbonyl, Cyano, Oximinomethyl, Formyl oder $C_2$ bis $C_3$ Alkoxycarbimidoyl darstellt,

mit einer Verbindung der allgemeinen Formel (III):

$$R_2-NH-R'_2 \qquad (III)$$

in welcher

$R_2$ die oben angegebene Bedeutung hat und $R'_2$ Wasserstoff oder Benzyl darstellt,

umsetzt, gegebenenfalls nachträglich in der erhaltenen Verbindung einen der Reste $R_1$, $R'_2$, $R'_3$ oder $R_4$ nach bekannten Methoden in einen anderen, durch den Anspruch 1 definierten Rest $R_1$, $R_3$ oder $R_4$ überführt, gewünschtenfalls ein racemisches Gemisch von Verbindungen der allgemeinen Formel (I) in an sich bekannter Weise in optisch aktive Formen trennt, und die erhaltenen Verbindungen gewünschtenfalls in ihre pharmakologisch verträgliche Salze überführt.

7. Aminopropanolderivate der allgemeinen Formel (I) sowie deren pharmakologisch verträgliche Salze zur Bekämpfung von Herz- und Kreislauferkrankungen sowie zu deren Prophylaxe.

8. Arzneimittel, enthaltend Verbindungen der allgemeinen Formel (I) oder deren pharmakologisch verträgliche Salze sowie an sich bekannte pharmakologisch verträgliche Trägerstoffe.

**Claims**

1. Aminopropanol derivates of the general formula (I):

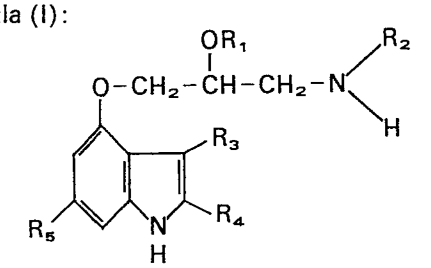

(I)

in which

$R_1$ signifies hydrogen, a $C_1$-$C_5$ alkanoyl or a benzoyl group,

$R_2$ a $C_1$-$C_6$ alkyl group or a group:

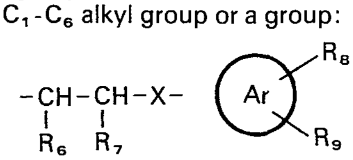

wherein

X signifies a bond, a methylene group or an oxygen atom,

Ar signifies a phenyl radical,

$R_6$ and $R_7$ are the same or different and each signifies hydrogen or a $C_1$-$C_6$ alkyl group,

$R_8$ and $R_9$ are the same or different and each signifies hydrogen or halogen, a hydroxyl group, a $C_1$-$C_5$ alkanoyl group, a $C_2$-$C_4$ alkenyl group, a $C_2$-$C_4$ alkynyl group, a $C_1$-$C_6$ alkyl group, a $C_1$-$C_6$ alkoxy group, a $C_2$-$C_4$ alkenyloxy group, a $C_2$-$C_4$ alkynyloxy group, a $C_1$-$C_6$ alkylthio group, an aminocarbonyl group, as well as an acetamido group,

$R_3$ signifies cyano,

$R_4$ signifies hydrogen, a $C_1$-$C_6$ alkyl group of a group $-CH_2-O-R_1$, whereby $R_1$ has the abovementioned meaning, and

$R_5$ signifies hydrogen or a $C_1$-$C_6$ alkyl group, their optically active forms, racemic mixtures, as well as their pharmacologically acceptable salts.

2. Compounds of general formula (I) according to claim 1, as well as their pharmacologically acceptable salts, in which $R_1$, $R_3$, $R_4$ and $R_5$ have the given meanings and $R_2$ signifies an isopropyl group.

3. Compounds of general formula (I) according to claim 1, as well as their pharmacologically acceptable salts, in which $R_1$, $R_3$, $R_4$ and $R_5$ have the given meanings and $R_2$ represents a group:

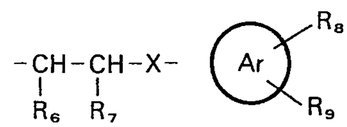

wherein

X signifies a bond, a methylene group or an oxygen atom,

Ar signifies a phenyl radical,

$R_6$ and $R_7$ are the same or different and each signifies hydrogen or a $C_1$-$C_6$ alkyl group,

$R_8$ and $R_9$ are the same or different and each signifies hydrogen or halogen, a hydroxyl group, a $C_1$-$C_5$ alkanoyl group, a $C_2$-$C_4$ alkenyl group, a $C_2$-$C_4$ alkynyl group, a $C_1$-$C_6$ alkyl group, a $C_1$-$C_6$ alkoxy group, a $C_2$-$C_4$ alkenyloxy group, a $C_2$-$C_4$ alkynyloxy group, a $C_1$-$C_6$ alkylthio group, an aminocarbonyl group, as well as an acetamido group.

4. Compounds of general formula (I) according to claim 3, as well as their pharmacologically acceptable salts, in which $R_6$, $R_7$, $R_8$, $R_9$ as well as Ar have the given meanings and X represents a valency bond.

5. Compounds of general formula (I) according to claim 3, as well as their pharmacologically acceptable salts, in which $R_6$, $R_7$, $R_8$, $R_9$ as well as Ar have the given meanings and X signifies an oxygen atom.

6. Process for the preparation of compounds of the general formula (I):

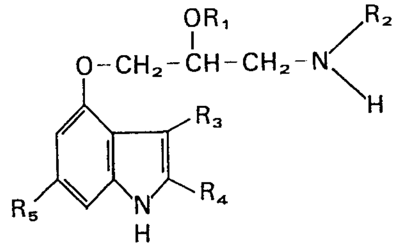

(I)

in which

$R_1$ signifies hydrogen, a $C_1$-$C_5$ alkanoyl or a benzoyl group,

$R_2$ a $C_1$-$C_6$ alkyl group or a group:

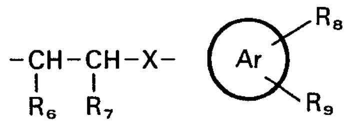

wherein

X signifies a bond, a methylene group or an oxygen atom,

Ar signifies a phenyl radical,

$R_6$ and $R_7$ are the same or different and each signifies hydrogen or a $C_1$-$C_6$ alkyl group,

$R_8$ and $R_9$ are the same or different and each signifies hydrogen or halogen, a hydroxyl group, a $C_1$-$C_5$ alkanoyl group, a $C_2$-$C_4$ alkenyl group, a $C_2$-$C_4$ alkynyl group, a $C_1$-$C_6$ alkyl group, a $C_1$-$C_6$ alkoxy group, a $C_2$-$C_4$ alkenyloxy group, a $C_2$-$C_4$ alkynyloxy group, a $C_1$-$C_6$ alkylthio group, an aminocarbonyl group, as well as an acetamido group,

$R_3$ signifies cyano,

$R_4$ signifies hydrogen, a $C_1$-$C_6$ alkyl group of a group $-CH_2-O-R_1$, whereby $R_1$ has the above-mentioned meaning, and

$R_5$ signifies hydrogen or a $C_1$-$C_6$ alkyl group, their optically active forms, racemic mixtures, as well as their pharmacologically acceptable salts, characterized in that, in per se known manner, one reacts a compound of the general formula (II):

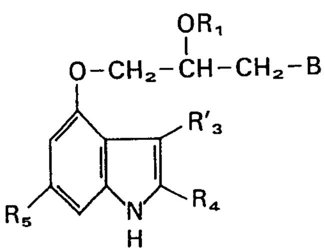

(II)

in which

$R_1$, $R_4$ and $R_5$ have the above-mentioned meaning,

B represents a reactive group or, together with $R_1$, a valency bond,

$R'_3$ signifies carboxyl, $C_2$-$C_3$ alkoxycarbonyl, aminocarbonyl, cyano, oximinomethyl, formyl or $C_2$-$C_3$ alkoxycarbimidoyl,

with a compound of the general formula (III):

$$R_2-NH-R'_2 \qquad (III)$$

in which

$R_2$ has the above-given meaning, and

$R'_2$ represents hydrogen or benzyl,

subsequently, in the compound obtained, one optionally converts one of the residues $R_1$, $R'_2$, $R'_3$ or $R_4$ according to known methods into another residue $R_1$, $R_3$ or $R_4$ defined by claim 1; if desired, one separates a racemic mixture of compounds of general formula (I) in per se known manner into optically active forms and, if desired, one converts the compounds obtained into their pharmacologically acceptable salts.

7. Aminopropanol derivatives of general formula (I), as well as their pharmacologically acceptable salts, for the combating of heart and circulatory diseases, as well as for their prophylaxis.

8. Medicaments containing compounds of general formula (I) or their pharmacologically acceptable salts, as well as per se known pharmacologically acceptable carrier materials.

**Revendications**

1. Dérivés d'aminopropanol de formule générale (I):

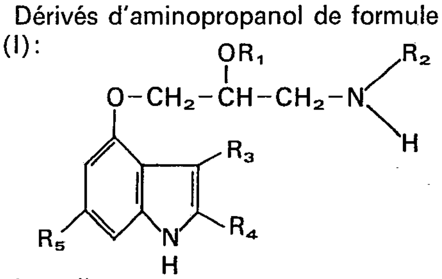

(I)

dans laquelle

$R_1$ est de l'hydrogène, un groupe alcanoyle $C_1$-$C_5$ ou un groupe benzoyle,

$R_2$ est un groupe alkyle $C_1$-$C_6$ ou un groupe:

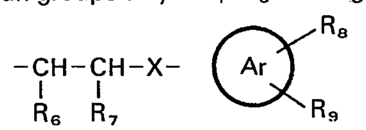

dans lequel

X est une liaison, un groupe méthylène ou un atome d'oxygène,

Ar est un reste phényle,

$R_6$ et $R_7$ sont, identiques ou différents, chacun de l'hydrogène ou un groupe alkyle $C_1$-$C_6$,

$R_8$ et $R_9$ sont, identiques ou différents, chacun de l'hydrogène ou de l'halogène, un groupe hydroxyde, un groupe alcanoyle $C_1$-$C_5$, un groupe alcényle $C_2$-$C_4$, un groupe alcynyle $C_2$-$C_4$, un groupe alkyle $C_1$-$C_6$, un groupe alcoxy $C_1$-$C_6$, un groupe alcényloxy $C_2$-$C_4$, un groupe alcynyloxy $C_2$-$C_4$, un groupe alkylthio $C_1$-$C_6$, un groupe aminocarbonyle, ou aussi un groupe acétamido,

$R_3$ est le groupe nitrile,

$R_4$ est de l'hydrogène, un groupe alkyle $C_1$-$C_6$ ou un groupe $-CH_2-O-R_1$ où $R_1$ a la signification donnée ci-dessus, et

$R_5$ est de l'hydrogène ou un groupe alkyle $C_1$-$C_6$,

leurs formes optiquement actives, leurs mélanges racémiques ainsi que leurs sels pharmacologiquement acceptables.

2. Composés de formule générale (I) selon la revendication 1, ainsi que leurs sels pharmacologiquement acceptables, dans lesquels $R_1$, $R_3$, $R_4$ et $R_5$ ont les significations indiquées et $R_2$ est un groupe isopropyle.

3. Composés de formule générale (I) selon la revendication 1, ainsi que leurs sels pharmacologiquement acceptables, dans lesquels $R_1$, $R_3$, $R_4$ et $R_5$ ont les significations indiquées et $R_2$ est un groupe:

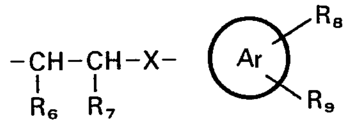

dans lequel

X est une liaison, un groupe méthylène ou un atome d'oxygène,

Ar est un reste phényle,

$R_6$ et $R_7$ sont, identiques ou différents, chacun de l'hydrogène ou un groupe alkyle $C_1$-$C_6$,

$R_8$ et $R_9$ sont, identiques ou différents, chacun de l'hydrogène ou de l'halogène, un groupe hydroxyde, un groupe alcanoyle $C_1$-$C_5$, un groupe alcényle $C_2$-$C_4$, un groupe alcynyle $C_2$-$C_4$, un groupe alkyle $C_1$-$C_6$, un groupe alcoxy $C_1$-$C_6$, un groupe alcényloxy $C_2$-$C_4$, un groupe alcynyloxy $C_2$-$C_4$, un groupe alkylthio $C_1$-$C_6$, un groupe aminocarbonyle, ou aussi un groupe acétamido.

4. Composés de formule générale (I) selon la revendication 3, ainsi que leurs sels pharmacologiquement acceptables, dans lesquels $R_6$, $R_7$, $R_8$, $R_9$ et Ar ont les significations indiquées et X est une liaison de valence.

5. Composés de formule générale (I) selon la revendication 3, ainsi que leurs sels pharmacologiquement acceptables, dans lesquels $R_6$, $R_7$, $R_8$, $R_9$ et Ar ont les significations indiquées et X est un atome d'oxygène.

6. Procédé pour la préparation de composés de formule générale (I):

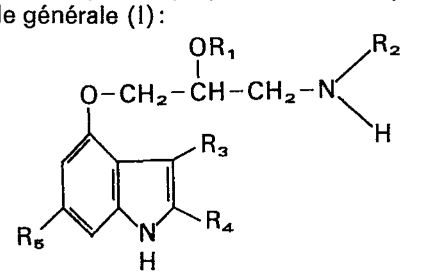

(I)

dans laquelle

$R_1$ est de l'hydrogène, un groupe alcanoyle $C_1$-$C_6$ ou un groupe benzoyle,

$R_2$ est un groupe alkyle $C_1$-$C_6$ ou un groupe:

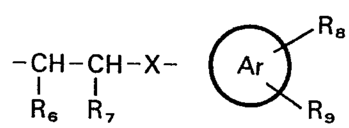

dans lequel

X est une liaison, un groupe méthylène ou un atome d'oxygène,

Ar est un reste phényle,

$R_6$ et $R_7$ sont, identiques ou différents, chacun de l'hydrogène ou un groupe alkyle $C_1$-$C_6$,

$R_8$ et $R_9$ sont, identiques ou différents, chacun de l'hydrogène ou de l'halogène, un groupe hydroxyde, un groupe alcanoyle $C_1$-$C_5$, un groupe alcényle $C_2$-$C_4$, un groupe alcynyle $C_2$-$C_4$, un groupe alkyle $C_1$-$C_6$, un groupe alcoxy $C_1$-$C_6$, un groupe alcényloxy $C_2$-$C_4$, un groupe alcynyloxy $C_2$-$C_4$, un groupe alkylthio $C_1$-$C_6$, un groupe aminocarbonyle, ou aussi un groupe acétamido,

$R_3$ est le groupe nitrile,

$R_4$ est de l'hydrogène, un groupe alkyle $C_1$-$C_6$ ou un groupe $-CH_2-O-R_1$ où $R_1$ a la signification donnée ci-dessus, et

$R_5$ est de l'hydrogène ou un groupe alkyle $C_1$-$C_6$,

leurs formes optiquement actives, leurs mélanges racémiques ainsi que leurs sels pharmacologiquement acceptables, caractérisé en ce que, de façon connue en soi on fait réagir un composé de formule générale (II):

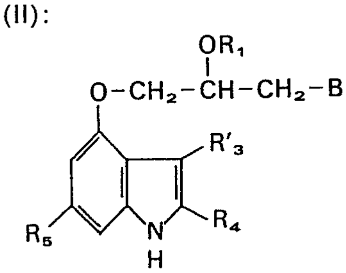

(II)

dans laquelle

$R_1$, $R_4$ et $R_5$ ont la signification ci-dessus indiquée,

B est un groupe réactif ou représente ensemble avec $R_1$ un trait de valence,

$R'_3$ représente carboxyle, alcoxycarbonyle $C_2$-$C_3$, aminocarbonyle, nitrile, oximinométhyle, formyle ou alcoxycarbimidoyle $C_2$-$C_3$,

avec un composé de formule générale (III):

$$R_2-NH-R'_2 \qquad (III)$$

dans laquelle

$R_2$ a la signification donnée ci-dessus, et $R'_2$ est de l'hydrogène ou du benzyle,

on transforme éventuellement ultérieurement dans le composé obtenu un des restes $R_1$, $R'_2$, $R'_3$ ou $R_4$, selon un procédé connu, en un autre reste $R_1$, $R_3$ ou $R_4$ défini dans la revendication 1; on sépare éventuellement un mélange racémique de composés de formule générale (I), de façon connue en soi, en formes optiquement actives, et on transforme éventuellement les composés obtenus en leurs sels pharmacologiquement acceptables.

7. Dérivés d'aminopropanol de formule générale (I), ainsi que leurs sels pharmacologiquement acceptables, pour le traitement et la prophylaxie des maladies cardiaques et circulatoires.

8. Médicament contenant des composés de formule générale (I) ou leurs sels pharmacologiquement acceptables, ainsi que des matières de support pharmacologiquement acceptables connues en soi.